Europäisches Patentamt

European Patent Office

Office européen des brevets

(11) Numéro de publication: **0 396 527**
**A1**

## (12) DEMANDE DE BREVET EUROPEEN

(21) Numéro de dépôt: **90870064.4**

(22) Date de dépôt: **02.05.90**

(51) Int. Cl.⁵: **A61K 7/48**

(30) Priorité: **03.05.89 BE 8900486**

(43) Date de publication de la demande:
**07.11.90 Bulletin 90/45**

(84) Etats contractants désignés:
**BE DE ES FR GB IT**

(71) Demandeur: **d'Oosterlynck, André**
**Museumlaan 17**
**B-9830 St. Martens Latem(BE)**

(72) Inventeur: **d'Oosterlynck, André**
**Museumlaan 17**
**B-9830 St. Martens Latem(BE)**

(74) Mandataire: **Pieraerts, Jacques et al**
**Bureau Gevers S.A. rue de Livourne 7 bte 1**
**B-1050 Bruxelles(BE)**

(54) Emulsion topique à propriétés régénératrices et curatives.

㊔ Emulsion topique à propriétés régénératrices et curatives applicable sur la peau, avec la caractéristique que le produit selon l'invention consiste en une émulsion eau dans huile, dont la phase huileuse ou fraction grasse, qui consiste en différents composants, présente une température de fusion de maximum 35 °C.

EP 0 396 527 A1

Xerox Copy Centre

## Emulsion topique à propriétés régénératrices et curatives applicable sur la peau

Cette invention concene une émulsion topique à propriétés régénératrices et curatives à appliquer sur la peau.

Il est bien connu que le sébum humain maintient la souplesse de l'épiderme et qu'il la protège contre les agressions, telles que par exemple le dessèchement. Un dérèglement de la quantité de sébum ou de sa composition provoque dans de nombreux cas des anomalies d'une peau dite "normale".

La présente invention vise à éliminer les inconvénients des crèmes pour la peau connues jusqu'à ce jour.

Pour procurer cette amélioration conformément à l'invention, le produit a été conçu comme une émulsion eau dans huile, dont la phase huileuse ou fraction grasse, qui consiste en différents composants, présente une température de fusion de maximum 35 ˚C.

Toujours selon l'invention, chacun des composants susdits de la phase huileuse a, quant à lui, une température de fusion inférieure à 35 ˚C et la phase huileuse susdite comprend un monoester, outre un émulsifiant et des composants actifs.

Le monoester susdit consiste dans une version avantageuse en cire liquide naturelle de jojoba, c.-à-d. de la cire obtenue de la noix de la plante *Simmondsia Chinensis (link) schneider.*

Dans une variante, le monoester susdit consiste en un érucate oléylique, c.-à-d. un monoester synthétique formé d'alcool oléylique et d'acide érucique.

Suivant une autre variante possible, le produit selon l'invention comprend, outre un émulsifiant et des composants actifs, un mélange de cire liquide naturelle de jojoba et d'érucate oléylique.

D'autres détails et avantages de l'invention ressortiront de la description suivante d'un produit topique selon l'invention à appliquer sur la peau. Cette description est uniquement donnée à titre d'exemple et ne limite pas l'invention.

La constatation que le sébum s'avère être également une émulsion eau dans huile est à la base de l'invention. La couche adipeuse de la peau n'est enlevée ni par lavage ni par rinçage à l'eau après l'évaporation de l'eau véhiculée. L'état naturel de l'épiderme est dès lors lipophile ou hydrophobe. La notion d'hydrophobie est incluse dans la notion de "lipophilie" ou d'affinité pour les graisses.

Il a également été constaté que l'application d'une émulsion du type huile dans eau, appelée également émulsion H/E, déstabilise l'hydrophobie de la peau ou les propriétés hydrofuges naturelles et qu'elle exerce, pour le moins, une influence nuisible à ce niveau. Pratiquement toutes les crèmes pour la peau actuellement commercialisées sont du type H/E.

L'influence nuisible sur la couche adipeuse protectrice de la peau est provoquée par la tendance de l'émulsifiant H/E à diluer ou à émulsionner le sébum par la diminution de la tension superficielle de la peau.

On peut dire en d'autres mots que l'émulsifiant utilisé est trop hydrophile et qu'il présente trop de propriétés de savon. Tout le monde sait que le savon nuit à la peau.

Les tentatives visant à améliorer ce caractère nuisible des émulsifiants H/E par la préparation d'une émulsion H/E avec des huiles et des graisses hautement hydrofuges ou hydrophobes sont peu efficaces, car elles n'éliminent pas la cause du mal.

Il a été constaté que les émulsions H/E sont pour le moins sans effet, alors que certaines compositions peuvent même être appelées agressives.

Que les produits dont il est question ici, c.-à-d. la plupart des crèmes pour la peau, soient constitués à base d'émulsions H/E est dû à au moins une des cinq causes nommées ci-après :

1. La production desdites émulsions ne pose pas beaucoup de problèmes. Pratiquement toutes les émulsions sont auto-émulsifiantes et la production de ces produits ou crèmes pour la peau peut se faire sans trop d'opérations mécaniques, telles qu'entre autres l'agitation.

2. La bonne conservabilité ou la possibilité de stockage desdites émulsions H/E, comparativement à celle des émulsions "eau dans huile", les émulsions dites E/H.

3. Le caractère trop gras des émulsions E/H produites et commercialisées jusqu'à aujourd'hui.

4. Les forces de mélange externes nettement supérieures nécessaires à la préparation d'émulsions E/H donnant des produits à conservabilité satisfaisante.

5. L'impossibilité constatée jusqu'à ce jour de préparer des émulsions E/H commerciales sans addition de matières grasses solides cireuses à la phase huileuse de l'émulsion. Les seuls produits commerciaux actuellement connus sont constitués à base d'émulsions E/H, qui présentent toutes une température de fusion de la phase huileuse qui se situe dans l'ordre de grandeur d'au moins 40 ˚C. Une telle température est de loin supérieure à la température de la peau humaine qui avoisine les 35 ˚C.

Il faut, avant de décrire en détail la structure du produit selon l'invention, expliquer ce qui suit sur le bilan hydrophile-lipophile (BHL) comme propriété principale des émulsifiants utilisés :

BHL zéro : en présence d'un tel BHL, on a à faire à une matière grasse hydrofuge. Dans ce cas limite, une telle substance ne peut pratiquement pas être considérée comme un émulsifiant. En voici des exemples : des esters végétaux et animaux, des huiles et des graisses minérales, ainsi que des esters cireux.

BHL > 0 à environ 6 à 7 :il s'agit ici d'émulsifiants non auto-émulsifiants à mal auto-émulsifiants. Ils ne sont pas hydrosolubles mais sont solubles dans de l'huile ou dans des matières grasses. Selon l'invention, il est uniquement fait usage ici d'émulsifiants qui font partie de ce groupe.

BHL > 6 à environ 9 à 10 : les émulsifiants qui font partie de ce groupe BHL ont tendance à former des émulsions H/E à mesure que croît la valeur du BHL. Comparativement à la propriété du sébum, il s'agit ici d'une émulsion dite "inverse". Il est possible, du moins théoriquement, de préparer de telles émulsions qui sont alors des types suivants : "huile dans eau dans huile" et vice versa, "eau dans huile dans eau". Ces formes théoriques d'émulsion ne sont pas appliquées selon l'invention et sont considérées comme peu pratiques, d'autant que les propriétés hydrofuges de la peau ne sont pas compatibles avec ce type d'émulsifiant.

BHL entre 9 et 15 : les émulsifiants qui font partie de ce groupe forment des émulsions H/E stables de façon très simple. Ces émulsions sont inutilisables dans le domaine de la dermatologie. Malgré ces propriétés, pratiquement tous les produits disponibles sur le marché sont préparés à base de telles émulsions.

BHL 14 à environ 20 : de tels émulsifiants donnent lieu uniquement à des gels H/E et à des microgels à mesure que croît la valeur croissante du BHL. Ces émulsifiants sont pratiquement hydrosolubles. De tels émulsifiants sont inutilisables dans le cadre de la présente invention.

BHL 20 à n : les produits de ce groupe forment des microgels lorsque le BHL est d'environ 20 et deviennent des microgels extrêmes à mesure que croît le BHL. A mesure que croissent les valeurs supérieures, on obtient des agents de surface, des détergents et des savons. Ces produits sont quasiment hydrosolubles à mesure que croît la valeur du BHL et le sont parfaitement pour les valeurs supérieures.

La raison pour laquelle des émulsions E/H conviennent parfaitement à une application topique ressortira ci-après de l'explication sur le bilan hydrophile-lipophile.

Dans le produit proposé par le demandeur il est uniquement fait usage d'un monoester liquide de jojoba qui peut être considéré comme un produit très bénéfique pour l'être humain. Ce monoester a satisfait à toutes les conditions visant à préserver les propriétés physiques du produit. Il s'ensuit que les propriétés de fluidité du monoester de jojobe sont entièrement respectées. Il ne faut donc pas ajouter d'adjuvants solidifiants, tels que des cires, des alcools gras et autres. Des agents volumisateurs minéraux, tels que des Bentones, des oxydes métalliques et autres, n'entrent pas en ligne de compte ici.

Les émulsifiants utilisés ont été sélectionnés comme expliqué ci-après :
- un BHL le plus bas possible. Dans la pratique il est utilisé un émulsifiant ayant un BHL situé entre 2 et 5 ;
- des propriétés allergènes les plus faibles possibles, c.-à-d. les propriétés les plus bénéfiques pour la peau.

Un choix d'excipients actifs a été opéré parmi les produits connus dans le domaine de la technique et appartenant aux oléo-vitamines et aux extraits végétaux éthériques.

La phase aqueuse de l'émulsion qui caractérise le produit présente uniquement des composants actifs. Les "régulateurs de l'humidité de la peau" connus jusqu'à présent s'avéraient peu efficaces à inefficaces. Des facteurs hydratants naturels, au contraire, ont été jugés acceptables, alors que les substances qui apparaissent toujours dans les produits topiques actuellement connus sont très nuisibles à la peau ; citons à titre d'exemple :

a) le glycérol et les dérivés à faible masse moléculaire ;

b) l'éthylèneglycol, le propylèneglycol ;

c) les sels de triéthanolamine et leurs composés ;

d) différents sels alcalins.

Des résultats positifs ont été obtenus avec des extraits de plantes et des vitamines dissous dans la phase aqueuse.

Des substances qui protègent le produit final contre les micro-organismes peuvent être utilisées.

Il s'est avéré qu'il était possible non seulement de produire une émulsion qui répondait aux exigences et aux normes posées, mais également de garantir la possibilité de stockage de l'émulsion.

Des expériences apprirent assez vite que des applications topiques du produit selon l'invention sur la peau humaine fournissent des résultats surprenants, notamment :

a) une sensation agréable lors de l'application, produit doux et facile à étaler ;

b) la constatation que l'émulsion est entièrement absorbée par la peau au bout de 3 à 5 minutes ;

c) la constatation également que la peau traitée présente un toucher velouté. Il a été constaté ici que la peau ne gardait aucune trace de graisse ;

d) que l'émulsion E/H ne puisse être enlevée de la peau par rinçage à l'eau est la cause du fait que le produit selon l'invention présente des propriétés de grande valeur. Une peau qui est fréquemment en contact avec de l'eau est parfaitement protégée par le produit selon l'invention. Des filtres solaires constitués à base de ce produit s'avéraient extrêmement efficaces.

On peut encore souligner, afin d'appuyer les propriétés du produit selon l'invention que la théorie encore toujours fort répandue, selon laquelle une émulsion E/H est un produit qui est gras, voire très gras au toucher, peut s'expliquer par la constatation qu'une recherche sur les produits en question a révélé que la température de fusion de la phase grasse utilisée jusqu'à présent est bien trop élevée. Cette température de fusion est nettement supérieure à la température de la peau humaine, à savoir supérieure à 35 °C. La phase grasse appartenant aux produits connus jusqu'à ce jour laissent sur la peau un film de graisse littéralement solidifié, ce qui explique l'effet gras au toucher.

De tels produits obturent les pores. Ils entraînent l'occlusion, ce qui doit être évité à tout prix. De telles substances donnent lieu à des comédons et autres.

L'émulsion H/E, qualifiée d'"émulsion inverse", qui présente généralement cette même phase grasse graisseuse solidifiée à 35 °C peut néanmoins être mieux étalée, parce que dans ce cas la phase stationnaire de l'émul sion est la phase aqueuse qui laisse sur la peau un film d'eau mouillant. La fraction grasse solidifiée d'une telle émulsion reste sur la peau sous la forme de petites particules de graisse qui n'obturent que partiellement la peau en raison de leurs dimensions, qui sont d'environ 1 micron. Il ne peut être escompté beaucoup d'effet d'une telle émulsion, du moins en ce qui concerne la phase grasse.

L'invention est donc essentiellement caractérisée par l'utilisation d'une émulsion "eau dans huile" qui ne présente pas les propriétés nuisibles de l'émulsion existante "eau dans huile".

Au contraire, le produit topique selon l'invention à appliquer sur la peau présente les propriétés suivantes :

- L'émulsion E/H s'étale facilement sur la peau et forme une petite couche d'huile qui reste toujours liquide. Cette couche pénètre facilement et rapidement dans l'épiderme. Aussi au bout de quelques minutes la peau est-elle "sèche" ou non grasse au toucher. La peau n'est jamais obturée (n'est pas occlue). Le produit selon l'invention ne donne dès lors jamais lieu à des comédons, ni à des inflammations.

- Les produits constitués à base d'une émulsion E/H règle les propriétés hydrofuges de la peau de façon naturelle. Après le traitement, la peau retrouve rapidement sa souplesse et sa douceur. Des peaux dites sèches se normalisent après quelques jours de traitement.

L'originalité du produit selon l'invention a été suivie par des dermatologues et les résultats des applications topiques ont été suivis avec attention ; il en est apparu que :

a) le produit peut être utilisé comme un cosmétique ;

b) le produit peut également être utilisé comme un moyen "curatif" régénérateur. Il a été remarqué à cet effet qu'en cas de couperose la coloration vive disparaissait et que les fissures des veinules s'estompaient après un traitement de quelques jours ;

c) la couche kératinisée devient moins épaisse, alors que l'épiderme s'épaissit jusqu'au triple et acquiert davantage de tonus. La régénération put être appelée spectaculaire au bout de quatre à six semaines de traitement. Les grandes "taches de vieillesse" d'un diamètre de plus de 3 mm s'estompaient, alors que les taches plus petites disparaissaient.

Il apparaissait également que le produit assurait d'excellents résultats dans le cas de certains maux, tels que le psoriasis, l'eczéma, l'acné, l'herpès labial, etc. Ces maux furent freinés de façon significative, sinon guéris.

Un cas particulièrement grave d'ulcère variqueux a été complètement et parfaitement guéri en quelques mois à l'aide du produit selon l'invention. Le traitement consistant en applications topiques.

**Revendications**

1. Emulsion topique à propriétés régénératrices et curatives à appliquer sur la peau, avec la caractéristique que le produit selon l'invention consiste en une émulsion eau dans huile, dont la phase huileuse ou fraction grasse, qui consiste en différents composants, présente une température de fusion de maximum 35 °C.

2. Le produit selon la conclusion 1, avec la caractéristique que chacun des composants susdits de la phase huileuse a, quant à lui, une température de fusion inférieure à 35 °C.

3. Le produit selon une des conclusions 1 ou 2, avec la caractéristique que les composants susdits sont sélectionnés de telle sorte à ce qu'ils soient compatibles entre eux et qu'ils se présentent comme un mélange homogène à une température de plus de 20 °C.

4. Le produit selon une des conclusions 2 ou 3, avec la caractéristique que la phase huileuse susdite comprend un monoester, outre un émulsifiant et des composants actifs.

5. Le produit selon la conclusion 4, avec la caractéristique que le monoester susdit consiste en cire liquide naturelle de jojobe, c.-à-d. de la cire obtenue de la noix de la plante *Simmondsia Chinensis (link) schneider.*

6. Le produit selon la conclusion 4, avec la caractéristique que le monoester susdit consiste en un érucate oléylique, c.-à-d. un monoester synthétique formé d'alcool oléylique et d'acide érucique.

7. Le produit selon la conclusion 4, avec la caractéristique qu'il comprend, outre un émulsifiant et des composants actifs, un mélange de cire liquide naturelle de jojobe et d'érucate oléylique.

8. Le produit selon une des conclusions 1 à 7, avec la caractéristique qu'il contient encore des vitamines et extraits de plantes oléosolubles.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | FR-A-2 509 989  (L'OREAL)<br>* Exemples 1,3-4,6-7; revendications * | 1-3,5,8 | A 61 K  7/48 |
| Y | FR-A-2 474 310  (L'OREAL)<br>* En entier * | 1-3,5,8 | |
| X | GB-A-2 109 233  (L'OREAL)<br>* Page 1, lignes 58-65; exemples 4,8,11-12 * | 1-3 | |
| X | EP-A-0 145 607  (L'OREAL)<br>* Page 3, lignes 7-16; exemples 5,6,10,13; revendications * | 1-3,8 | |
| X | DE-C-3 725 139  (RIEKE)<br>* Revendications; exemples; page 4, ligne 68 - page 5, ligne 1 * | 1,2,8 | |
| A | EP-A-0 069 946  (C.I.R.T.A.)<br>* En entier * | 1-8 | |

DOMAINES TECHNIQUES
RECHERCHES (Int. Cl.5)

A 61 K

Le présent rapport a été établi pour toutes les revendications

| Lieu de la recherche | Date d'achèvement de la recherche | Examinateur |
|---|---|---|
| LA HAYE | 24-08-1990 | FISCHER J.P. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
.............................................................
& : membre de la même famille, document correspondant

EPO FORM 1503 03.82 (P0402)